# EUROPÄISCHE PATENTANMELDUNG

(11) **EP 1 862 139 A2**
(43) Veröffentlichungstag der Anmeldung: **05.12.2007**
(21) Anmeldenummer: 07075546.7
(22) Anmeldetag: 20.05.2003
(51) Int. Cl.: A61B 18/14, A61N 1/06, A61N 1/05

(54) **Therapiegerät**

(30) Priorität: 27.05.2002 DE 10224153
(62) Teilanmeldung aus: 03730081.1
(71) Anmelder: Celon AG Medical Instruments, 14513 Teltow (DE)
(72) Erfinder: Desinger, Kai, 12157 Berlin (DE); Fay, Markus, 14513 Teltow (DE); Roggan, André, 12163 Berlin (DE); Rothe, Rainer, 33165 Lichtenau (DE)
(74) Vertreter: Eisenführ, Speiser & Partner

(57) **Zusammenfassung**

Applikationsanordnung zum Applizieren eines Hochfrequenzstroms zur thermischen Verödung von Körpergewebe, umfassend: eine Elektrodennadel (1) mit einem elektrisch leitenden Schaft (3), mindestens einen den elektrisch leitenden Schaft (3) umgebenden und relativ zum Schaft (3) verschiebbaren isolierenden Hüllkörper (7) mit einem distalen Ende, aus dem der Schaft (3) ausfahrbar ist, wobei an der Aussenseite des isolierenden Hüllkörpers (7) eine Gegenelektrode (25) angeordnet ist.

## Beschreibung

Die Erfindung betrifft ein Therapiegerät, insbesondere eine Applikationsanordnung zum Applizieren eines Hochfrequenzstroms zur thermischen Verödung von Körpergewebe.

Das elektrochirurgische, insbesondere das elektrothermische Veröden von pathologisch verändertem Körpergewebe ist eine in der Medizin bekannte Methode. Von besonderem Interesse ist diese Methode für die Therapie von Organtumoren, z. B. Lebertumoren. Zur Verödung werden eine oder mehrere Elektroden im zu verödenden Gewebe, d. h. dem Tumorgewebe, oder in dessen unmittelbarer Nähe platziert und ein Wechselstrom zwischen den Elektroden oder einer Elektrode und einer außen am Körper fixierten, sogenannten Neutralelektrode fließen lassen. Fließt der Strom zwischen der Elektrode und der Neutralelektrode (ggf. auch zwischen mehreren Elektroden und einer oder mehreren Neutralelektroden), so spricht man von einer monopolaren Elektrodenanordnung. Fließt der Strom dagegen zwischen den im Gewebe befindlichen Elektroden selbst (in diesem Fall müssen mindestens zwei Elektroden in das Gewebe eingebracht werden), so spricht man von einer bipolaren Anordnung.

Zum Veröden des pathologisch veränderten Gewebes wird mittels Hochfrequenzgenerator ein Stromfluss zwischen den mit dem Körpergewebe in elektrisch leitendem Kontakt stehenden, sog. aktiven Elektroden und beispielsweise einer Neutralelektrode induziert. Der elektrische Widerstand des Körpergewebes führt dabei dazu, dass der Wechselstrom in Wärme umgewandelt wird. Bei Temperaturen zwischen 50° C und 100° C kommt es zu einer massiven Denaturierung der körpereigenen Proteine und in der Folge zum Absterben der betroffenen Gewebeareale. Aufgrund der hohen Stromdichte im Bereich der aktiven Elektroden erfolgt die Erwärmung des Gewebes vorwiegend dort, wo die aktiven Elektroden mit dem Körpergewebe im elektrisch leitfähigen Kontakt stehen.

Entscheidend für eine effektive und vor allem sichere Therapie ist die Ausbildung einer thermischen Destruktionszone, die an die Ausdehnung des pathologischen Gewebes optimal angepasst ist. Hier spielt die Länge des nicht isolierten aktiven Bereichs der Elektrodennadel eine entscheidende Rolle. Je länger dieser Bereich ist, desto größer ist die axiale Ausdehnung der thermischen Destruktionszone.

Die zum Platzieren im Gewebe vorgesehene Elektrode ist in der Regel auf einer Elektrodennadel angeordnet. Eine Elektrodennadel ist beispielsweise in US 2002/0035363 beschrieben. Die darin beschriebene Elektrodennadel umfasst einen elektrisch leitenden Schaft und einen relativ zum Schaft axial verschiebbaren isolierenden Mantel. Durch Verschieben des isolierenden Mantels lässt sich die aktive Oberfläche, d. h. die für die Anwendung der Elektrodennadel mit dem Körpergewebe in Kontakt zu bringende Oberfläche des Schaftes festlegen.

Die US 2002/0035363 beschreibt außerdem einen Trokar, durch den die Elektrodennadel in Körpergewebe einführbar ist. Ein Trokar ist eine Körpersonde mit einem zum Einführen in Körpergewebe vorgesehenen und einem zum Verbleib außerhalb des Körpergewebes vorgesehenen Abschnitt sowie einem freien Lumen zum Einführen von Instrumenten oder zum Ein- oder Ausleiten von Flüssigkeiten. Er findet beispielsweise Verwendung, um Flüssigkeiten aus Körperhöhlen zu entleeren oder Medikamente gezielt in bestimmte Körperregionen einzuführen.

Aufgabe der Erfindung ist, es eine alternative Applikationsanordnung mit einem Schaft und einem axial gegen den Schaft verschiebbaren Hüllkörper zu schaffen, die einen einfachen Aufbau aufweist und
die flexibel einsetzbar ist.

Die Aufgabe wird durch eine Applikationsanordnung nach Anspruch 1 gelöst.

Gemäß Anspruch 1 wird zum Lösen der zweiten Aufgabe eine Applikationsanordnung zum Applizieren eines Hochfrequenzstromes zur thermischen Verödung von Körpergewebe zur Verfügung gestellt, die umfasst:
- eine Elektrodennadel mit einem elektrisch leitenden Schaft und
- mindestens einen den elektrischen leitenden Schaft umgebenden und relativ zum Schaft verschiebbaren isolierenden Hüllkörper mit einem distalen Ende, aus dem der Schaft ausfahrbar ist.

Die Applikationsanordnung nach Anspruch 1 zeichnet sich dadurch aus, dass an der Außenseite des isolierenden Hüllkörpers eine Gegenelektrode angeordnet ist. Als Gegenelektrode soll hierbei jede Elektrode zu verstehen sein, die den bipolaren Betrieb der Applikationsvorrichtung ermöglicht. Insbesondere sind der Schaft und die Gegenelektrode elektrisch unabhängig voneinander ausgestaltet, d. h. an sie können jeweils unabhängig voneinander elektrische Potentiale, insbesondere von einem Hochfrequenzgenerator erzeugte elektrische Potentiale, angelegt werden, so dass zwischen ihnen ein Hochfrequenzstrom fließt.

Eine derartige Elektrodennadel lässt sich sowohl bipolar als auch monopolar betreiben. Die Verschiebbarkeit des Hüllkörpers ermöglicht es, den Stromfluss durch das zu behandelnde Körpergewebe zu beeinflussen, indem die Länge des aus dem Hüllkörper vorstehenden Schaftabschnittes der Elektrodennadel - und damit die als Elektrode mit dem Körpergewebe in elektrisch leitenden Kontakt bringbare wirksame Schaftoberfläche - variiert wird.

Die beschriebene Gegenelektrode kann auch dann Verwendung finden, wenn der Hüllkörper von einem Trokar gebildet ist. Sie befindet sich dann auf dem zum Einführen in Körpergewebe vorgesehenen Abschnitt des Trokars. Vorteilhafterweise weist der Trokar dann einen eigenen elektrischen Anschluss zum Anschließen eines Hochfrequenzgenerators an die Gegenelektrode auf. Der elektrische Anschluss kann dabei als Steckkontakt ausgebildet sein, zu dem ein Gegenstück an einem nicht zum Einbringen in das Körpergewebe vorgesehen Teil der Elektrodennadel existiert, so dass der Trokar über die Elektrodennadel mit dem Hochfrequenzgenerator zu verbinden ist. Besonders Anwenderfreundlich ist es, wenn der Steckkontakt und das Gegenstück derart angeordnet und ausgestaltet sind, dass die Verbindung des Steckkontaktes mit dem Gegenstück beim Einführen der Elektrodennadel in den Trokar selbständig zustande kommt.

Die Applikationsanordnung nach Anspruch 1 zeichnet sich dadurch aus, dass der zum Einführen in Körpergewebe vorgesehene Abschnitt des Trokars elektrisch isolierend ausgebildet ist und, insbesondere bei durch das Lumen hindurchgeführter Elektrodennadel bzw. hindurchgeführtem Schaft, den Hüllkörper für den Schaft der Elektrodennadel bildet.

Der Trokar erfüllt zwei Funktionen. Zum einen erfüllt er die herkömmlichen Aufgaben eines Trokars, nämlich beispielsweise die zielgenaue Medikamentenzuführung oder Gewebeentnahme sowie das Einführen von Elektrodennadeln zu ermöglichen, ohne dass jedes Mal neu punktiert werden muss. Zum anderen dient der im Körper befindliche Teil des Trokars als relativ zum Schaft der Elektrodennadel verschiebbarer isolierender Hüllkörper zum Einstellen der Länge des aktiven Bereiches des Schaftes, d. h. desjenigen Bereiches, der aus dem isolierenden Hüllkörper herausragt und bei in den Körper eingeführter Elektrodennadel mit dem Körpergewebe in elektrisch leitendem Kontakt steht. Eine derartige Applikationsanordnung weist gegenüber dem Stand der Technik, in dem zusätzlich zum Trokar eine verschiebbare isolierende Hülle um den Schaft der Elektrodennadel vorhanden ist, einen vereinfachten Aufbau auf. Insbesondere ist mit der erfindungsgemäßen Applikationsanordnung auch die Länge des aktiven Bereiches von Elektrodennadeln, die nicht mit einer eigenen isolierenden Hülle ausgestattet sind, einstellbar.

Weil der Trokar den Hüllkörper der Elektrodennadel bildet und diese daher keinen eigenen Hüllkörper benötigt, kann der Gesamtdurchmesser des zum Einführen in das Körpergewebe vorgesehenen Abschnitts des Trokars gering gehalten werde. Das Punktieren mit der erfindungsgemäßen Applikationsanordnung ist daher weniger traumatisch als mit der Trokar-Nadel-Kombination nach Stand der Technik.

Zum Verschieben des Schaftes relativ zum Hüllkörper, d. h. zum Trokar, ist eine Verschiebeeinrichtung, bspw. unter Verwendung eines Führungselementes, vorhanden, mit der sich die Länge des aus dem distalen Ende des Hüllkörpers herausragenden Teils des Schaftes einstellen lässt. Die Verschiebeeinrichtung kann insbesondere einen Klemm- oder Schraubmechanismus zum Arretieren der Elektrodennadel relativ zum Hüllkörper umfassen, um einem ungewollten Verschieben des Hüllkörpers relativ zur Elektrodennadel entgegen zu wirken.

Ein besonders genaues Einstellen der Länge des aus dem distalen Ende des Hüllkörpers herausragenden Teils des Schaftes lässt sich dadurch erzielen, dass die Applikationsanordnung an der Elektrodennadel, insbesondere am proximalen Ende des Schaftes, ein Führungselement aufweist und zum axialen Verschieben des Hüllkörpers relativ zum Schaft eine Innen- und Außengewindekombination am Trokar und am Führungselement vorhanden ist. Die Innen- und Außengewindekombination ermöglicht es, durch Drehen des Führungselementes relativ zum Trokar, den Hüllkörper gegenüber dem Schaft präzise zu verschieben. Durch die geeignete Wahl der Gewindesteigung lässt sich die Genauigkeit der Feineinstellung der Verschiebung festlegen. Je geringer die Gewindesteigung ist, je geringer fällt die Verschiebung z. B. bei einer vollen Gewindedrehung aus, d. h. desto genauer kann die Feineinstellung vorgenommen werden.

Um ein definiertes Einstellen der Länge des aus dem Hüllkörper herausragenden Teils des Schaftes zu ermöglichen, können die Elektrodennadel oder der Trokar, insbesondere der Klemm- oder Schraubmechanismus, Markierungen umfassen, aus denen sich die Länge des aus dem distalen Ende des Hüllkörpers herausragenden Teils des Schaftes bei in dem Körper eingeführten Schaft entnehmen lässt. Die Markierungen ermöglichen das gezielte Einstellen der aktiven Länge der Elektrode auch bei in den Körper eingeführter Elektrodennadel.

In einer Ausgestaltung der Erfindung zeichnet sich der Hüllkörper dadurch aus, dass er den Schaft eng umschließt. Das enge Umschließen verhindert, dass Körperflüssigkeit zwischen den Schaft und den Hüllmantel eindringt. Eindringende Körperflüssigkeiten könnten als leitende Flüssigkeit dazu führen, dass nicht nur aus dem Hüllmantel vorstehende und daher nicht abgedeckte Bereich des Schaftes mit dem Körpergewebe in elektrisch leitfähiger Verbindung steht, sondern auch Bereiche des Schaftes, die eigentlich gegen das Körpergewebe elektrisch isoliert sein sollten.

In einer weiteren Ausgestaltung der Erfindung ist zwischen dem Schaft und der Innenseite des Hüllkörpers ein Spalt vorhanden. Außerdem kann der zum Verbleib außerhalb des Körpers vorgesehene Abschnitt des Trokars eine Flüssigkeitszuführung zum Zuführen von Fluiden in den Spalt aufweisen. Der Spalt ermöglicht es, bei eingeführter Elektrodennadel Fluide, insbesondere Flüssigkeiten, in das Zielgebiet des Körpergewebes einzubringen. Flüssigkeiten, die in das Zielgebiet eingebracht werden können, sind beispielsweise Medikamente, Schmerzmittel, Spülmittel oder Flüssigkeiten, die dem Austrocknen des Gewebes während der Applikation des Hochfrequenzstroms entgegenwirken und so die Effizienz der thermischen Destruktion erheblich steigern. Im letzteren Falle sind die Flüssigkeiten vorzugsweise elektrisch leitfähig, um den elektrischen Kontakt des Schaftes mit dem Körpergewebe Aufrecht zu erhalten. Als elektrisch leitfähige Flüssigkeiten bieten sich z. B. physiologische Kochsalzlösungen an.

Die Abmessung des Spaltes kann vorteilhaft derart gewählt sein, dass ein bestimmter Flüssigkeitsdruck überschritten werden muss, damit die Flüssigkeit durch den Spalt fließen kann. Durch diese Ausgestaltung lässt sich verhindern, dass elektrisch leitende Körperflüssigkeiten in den Spalt eindringen und so eine elektrisch leitfähige Verbindung zwischen dem Körpergewebe und Teilen des Schaftes herstellen, die eigentlich gegen das Körpergewebe durch den Hüllkörper isoliert sein sollten.

Eine weitere Ausgestaltung der Erfindung zeichnet sich dadurch aus, dass der Schaft an seinem distalen Ende mit einer Spitze versehen ist. Die Spitze, die bei in den Hüllkörper eingeführtem Schaft aus dem distalen Ende des Hüllkörpers herausragt, kann beim Einführen des Trokars in das Körpergewebe als Punktiermittel dienen, so dass die Elektrodennadel als Einführhilfe für den Trokar Verwendung finden kann.

Um das Einführen der Applikationsvorrichtung und insbesondere des Übergangs zwischen dem Schaft und dem Hüllkörper in das Körpergewebe zu vereinfachen, kann der Hüllkörper an seinem distalen Ende eine Phase, d.h. einen sich verjüngenden Abschnitt aufweisen, so dass kein stufiger Übergang zwischen dem Hüllkörper und dem Schaft vorhanden ist.

In einer vorteilhaften Ausgestaltung der Applikationsanordnung umfasst der zum Einführen in Körpergewebe vorgesehene Abschnitt des Trokars ein Material, das ihn in einer computertomographischen oder kernspintomographischen Aufnahme sichtbar macht, so dass der Sitz des Trokars mittels Computertomographie oder Kernspintomographie kontrolliert werden kann. Ein solches Material kann beispielsweise Gold sein.

Weitere Merkmale und Vorteile der Erfindung werden nachfolgend anhand der Beschreibung von Ausführungsbeispielen unter Bezugnahme auf die beiliegenden Zeichnungen beschrieben.
- Figur 1: zeigt in perspektivischer Darstellung die Elektrodennadel und den Trokar einer Ausführungsform der erfinderischen Applikationsanordnung.
- Figur 2: zeigt das Ausführungsbeispiel aus Figur 1 mit in den Trokar eingeführter Elektrodennadel in perspektivischer Darstellung
- Figur 3: zeigt die Ausführungsform der erfinderischen Applikationsanordnung im Längsschnitt.
- Figur 4: zeigt einen Querschnitt durch Hüllkörper und Schaft einer zweiten Ausführungsform der erfinderischen Applikationsanordnung.
- Figur 5: zeigt einen Querschnitt durch Hüllkörper und Schaft einer dritten Ausführungsform der erfinderischen Applikationsvorrichtung.
- Figur 6: zeigt eine erste Behandlungskonfiguration unter Verwendung der erfindungsgemäßen Applikationsanordnung.
- Figur 7: zeigt eine zweite Behandlungskonfiguration unter Verwendung der erfindungsgemäßen Applikationsanordnung.
- Figuren 8A - 8C: zeigen ein Beispiel für eine medizinische Behandlung mit der erfindungsgemäßen Applikationsanordnung.

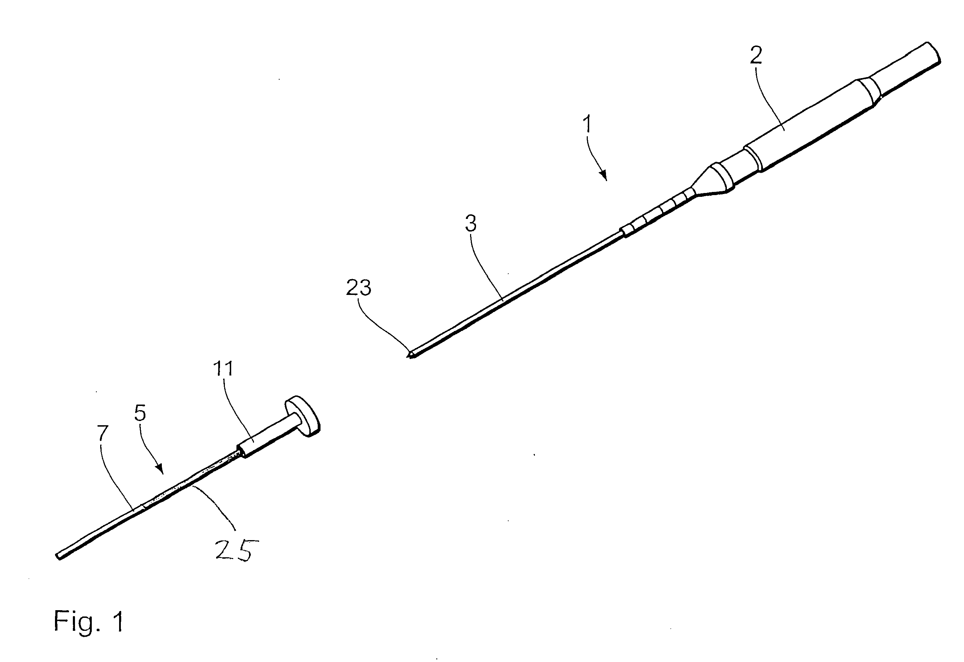

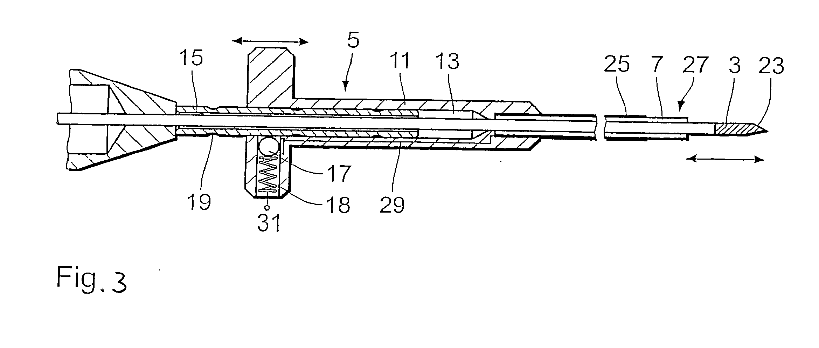

In Figur 1 ist ein Ausführungsbeispiel für die erfindungsgemäße Applikationsanordnung in perspektivischer Ansicht dargestellt. Die Applikationsanordnung umfasst eine Elektrodennadel 1 mit einem Griffabschnitt 2 zum Handhaben der Elektrodennadel 1 und einem Schaft 3 aus einem elektrisch leitfähigen Material, der mit einem nicht dargestellten Hochfrequenzgenerator verbunden werden kann. Sie umfasst weiterhin einen Trokar 5 mit einem zum Einführen in Körpergewebe ausgebildeten Abschnitt 7 und einen zum Verbleib außerhalb des Körpergewebes ausgebildeten Abschnitt 11. Der Trokar 5 weist ein Lumen 13 (siehe Fig. 3) auf, durch das die Elektrodennadel 1 derart einzuführen ist, dass sich der Schaft 3 der Elektrodennadel 1 durch den Abschnitt 7 des Trokars 5 erstreckt.

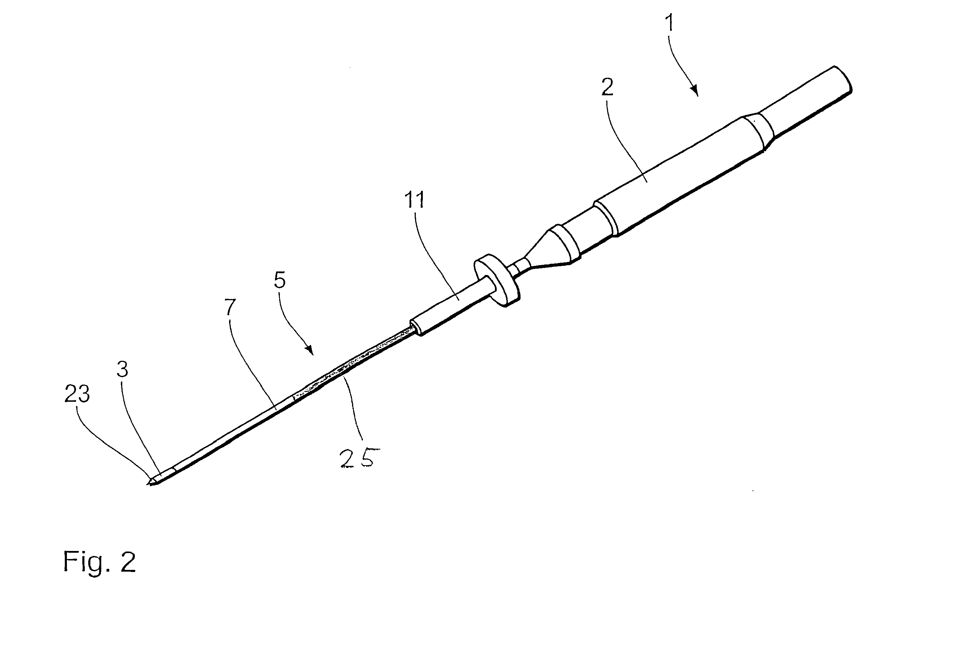

Ist die Elektrodennadel 1 in den Trokar 5 eingeführt (Figur 2), umschließt der Abschnitt 7 den Schaft 3 der Elektrodennadel 1 eng. Er besteht aus einem isolierenden Material, so dass er einen isolierenden Hüllkörper für den Schaft 3 der Elektrodennadel 1 bildet.

Mittels einer Verschiebeeinrichtung, die im in Fig. 3 dargestellten Ausführungsbeispiel ein zylindrisches Führungselement 15 umfasst, lässt sich die durch das Lumen 13 des Trokars 5 eingeführte Elektrodennadel 1 axial relativ zum Trokar 5 verschieben. Die Verschiebeeinrichtung umfasst dabei Markierungen, die im vorliegenden Ausführungsbeispiel als sich um den Umfang des Führungselements15 erstreckende Ringnuten 19 ausgebildet sind. Am Trokar 5 ist ein mit der Verschiebeeinrichtung 15 zusammenwirkender Klemmmechanismus 17 in Form einer zum Eingriff in die Ringnuten 19 ausgebildeten Kugel vorhanden. Die Kugel 17 wird durch eine vorgespannte Druckfeder 18 gegen die Umfangsfläche der Verschiebeeinrichtung gepresst und kann in die Ringnuten 19 einrasten, um die Verschiebeeinrichtung so gegen ein ungewolltes axiales Verschieben zu sichern. Statt der Ringnuten 19 können auch andere Arretiermittel Verwendung finden, sofern sie ein Einrasten der Kugel 17 ermöglichen.

Durch das Verschieben der Elektrodennadel 1 mittels des Führungselements 15 relativ zum Trokar 5, und damit zum Hüllkörper 7, lässt sich die Länge des aus dem Hüllkörper 7 vorstehenden distalen Abschnitts des elektrisch leitenden Schaftes 3 variieren. Dabei bildet der aus dem distalen Ende des Hüllkörpers 7 vorstehende Abschnitt des Schaftes 3 die aktive Elektrode der Elektrodennadel 1, d. h. die nach dem Einbringen in das Körpergewebe mit diesem in Kontakt stehende aktive Elektrode.

Über eine nicht dargestellte, im Inneren des Schaftes 3 verlaufende Leitung ist dieser mit einem Hochfrequenzgenerator verbunden durch den an die aktive Elektrode eine Hochfrequenzspannung anlegbar ist. Beim Applizieren der Hochfrequenzspannung ist an der Außenseite des Körpers eine Gegenelektrode angelegt, so dass zwischen dem Schaft 3 und der Gegenelektrode ein Hochfrequenzstrom fließen kann, der zur Destruktion des Körpergewebes, bspw. eines Tumorgewebes, führt. Die Form und Größe der Destruktionszone lässt sich dabei durch die Länge des aus dem distalen Ende des Hüllkörpers 7 vorstehenden Abschnittes des Schaftes 3 variieren.

Der Schaft 3 der Elektrodennadel 1 kann darüber hinaus auch zum Einführen des Trokars 5 in das Körpergewebe verwendet werden. Dazu weist der Schaft 3 an seinem distalen Ende eine Spitze 23 zum Punktieren des Körpergewebes auf.

Nach der Applikation des Hochfrequenzstromes kann der Trokar 5 im Körpergewebe verbleiben, wobei dann lediglich die Elektrodennadel 1 aus dem Trokar 5 herausgezogen wird. Er kann dann z.B. zum Einbringen von Medikamenten Verwendung finden. Der Hüllkörper 7 verhindert ein Verschleppen von Tumorzellen beim Herausziehen der Elektrodennadel.

Wird der Trokar 5 nach dem Applizieren des Hochfrequenzstromes oder ggf. später aus dem Gewebe herausgezogen, kann beim Herausziehen ein Fibrinkleber in den Penetrationskanal eingeführt werden, um ihn zu versiegeln.

Die Applikationsanordnung umfasst eine weitere, zweite Elektrode 25, die als Gegenelektrode zur durch den Schaft 3 gebildeten Schaftelektrode dient. Sie ist auf dem äußeren Umfang des elektrisch isolierenden Hüllkörpers 7 angeordnet. Die axiale Länge der Gegenelektrode 25 beträgt etwa das 1-20-fache des Durchmessers des Schaftes 3.

Am distalen Ende des Hüllkörpers 7 erstreckt sich um den gesamten Umfang des Hüllkörpers 7 herum ein isolierender Abschnitt 27, der die Gegenelektrode 25 vom Schaft 3 isoliert und den Abstand der Gegenelektrode 25 von der Schaftelektrode festlegt. Die Gegenelektrode 25 weist eine vom Schaft 3 getrennte, durch den Mantel des Abschnittes 11 des Trokars 5 verlaufende elektrische Zuleitung 29 auf, die über die Kugel 17, im vorliegenden Ausführungsbeispiel eine elektrisch leitfähige Kugel, beispielsweise eine Metallkugel, und die Druckfeder 18 mit einem Anschluss 31 zum Anschließen eines Hochfrequenzgenerators verbunden ist. Mit dieser Ausgestaltung ist der bipolare Betrieb der Applikationsanordnung möglich.

Im in Fig. 3 beschriebenen Ausführungsbeispiel kann der Hüllmantel 7 anstatt als Bestandteil des Trokars 5 auch als Bestandteil der Elektrodennadel 1 ausgebildet sein, wobei der Hüllmantel 7 weiterhin verschiebbar gegen den Schaft 3 ausgebildet ist. Eine derartig ausgestaltete Elektrodennadel kann auch ohne Trokar Verwendung finden. Das Vorhandensein eines Trokars ist daher zum Variieren der Länge des aus dem Hüllmantel 7 herausragenden Teils des Schaftes 3 nicht nötig.

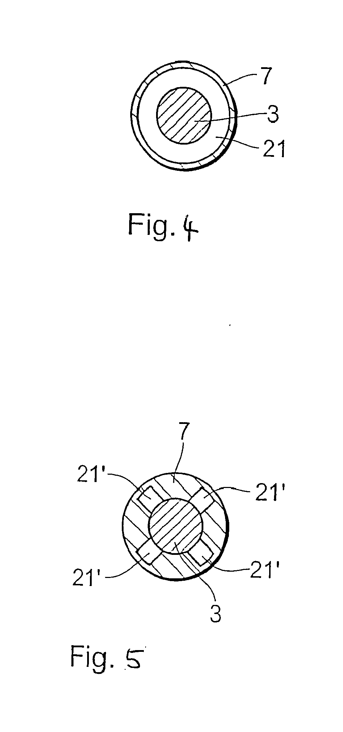

Weitere Ausführungsformen der erfindungsgemäßen Applikationsanordnung sind in den Figuren 4 und 5 dargestellt, die einen Querschnitt durch den Hüllkörper 7 und den in das Lumen des Hüllkörpers 7 eingeführten Schaft 3 darstellen. Im in Figur 5 dargestellten Ausführungsbeispiel erstreckt sich zwischen der Innenwand des Hüllkörpers 7 und der Außenfläche des Schaftes 3 ein Ringspalt 21, durch den beispielsweise eine Spülflüssigkeit in das zu behandelnde Gewebe eingeführt werden kann. Zudem ermöglicht es dieser Spalt 21 bei der Behandlung entstehenden Gasen aus dem Körper auszutreten.

Eine alternative Ausgestaltung ist in Figur 5 dargestellt. Statt einem Spalt, der sich um den gesamten Umfang des Schaftes 3 erstreckt, sind in diesem Ausführungsbeispiel vier Spalte 21', die sich jeweils nur über einen Teil des Umfanges des Schaftes 3 erstrecken, ausgebildet. Sie sind als in axialer Richtung verlaufende Kerben an der Innenfläche des Hüllkörpers 7 ausgebildet und erstrecken sich über dessen gesamte Länge. Selbstverständlich kann die Zahl der Kerben auch größer oder kleiner als vier sein. Die Kerben müssen auch keinen viereckigen Querschnitt aufweisen sondern können beispielsweise auch einen dreieckigen oder rundlichen Querschnitt aufweisen.

Die in Figur 5 dargestellte Ausgestaltung ermöglicht es, dass der Hüllkörper 7 den Schaft 3 eng umschließt und somit durch seine Reibung einem Verschieben des Schaftes gegen den Hüllkörper 7 entgegen wirkt. Gleichzeitig können jedoch der zu behandelnden Stelle im Körper durch die Spalte 21' Flüssigkeiten zugeführt werden. Ebenso können bei der Behandlung entstehende Gase entweichen. Die Abmessungen der Kerben 21' sind vorzugsweise so gewählt, dass ein vorbestimmter Flüssigkeitsdruck überschritten werden muss, damit die Flüssigkeit durch die Kerben 21' fließen kann. Körperflüssigkeiten, die zumeist elektrisch leitfähig sind, können dann nicht ohne weiteres in die Kerben 21' eindringen.

Zum Einführen von Flüssigkeiten in das Körpergewebe kann der Trokar an seinem zum Verbleib außerhalb des Körpers ausgebildeten Abschnitt 11 eine Phase für einen Spritzenanschluss aufweisen. Statt dem oder zusätzlich zum Spritzenanschluss kann der Trokar auch eine seitliche Flüssigkeitszuführung aufweisen.

Die Elektrodennadel 1 und/oder der zum Verbleib außerhalb des Körpers vorgesehene Abschnitt 11 des Trokars können außerdem mit Markierungen versehen sein, die dem Anwender zeigen, wie weit der Schaft 3 aus dem Hüllkörper 7 herausragt. Dies ist insbesondere wichtig, wenn die Applikationsanordnung bereits im Körper angeordnet ist, so dass der Anwender keinen visuellen Kontakt mit dem distalen Ende des Hüllkörpers 7 hat. Als solche Markierungen können beispielsweise die am Führungselement 15 angeordneten Ringnuten 19, farbige Markierungen oder eine Kombination aus beidem dienen.

Zum Vereinfachen des Punktierens kann der Hüllkörper 7 an seinem distalen Ende außerdem eine Phase aufweisen, die in den Figuren nicht dargestellt ist.

Der Trokar 5 kann außerdem in einer alternativen Ausführungsform gegen das Austreten von Gasen und Körperflüssigkeiten abgedichtet sein. In diesem Fall kann die Dichtung beispielsweise als ringförmige Dichtung am Innenumfang des zum Verbleib außerhalb des Körpers vorgesehenen Abschnittes 11 angeordnet sein und derart ausgebildet sein, dass sie in die Ringnuten 19 des Führungselementes 15 einrasten kann, so dass die Dichtung gleichzeitig als Rastelement zum Arretieren der Elektrodennadel in einer axialen Position relativ zum Trokar zu verwenden ist. Die Dichtung kann alternativ auch am Führungselement angeordnet sein, wobei die Ringnuten dann an der Innenfläche des Abschnittes 11 angeordnet sind.

Zur besseren Handhabung ist es insbesondere günstig, wenn der Trokar 5 und/oder der Griffabschnitt 2 der Elektrodennadel 1 eine Riffelung aufweisen.

In einer weiteren Ausgestaltung der Erfindung kann der Trokar mehrere achsenparallele, zum Einführen in Körpergewebe vorgesehene Abschnitte als Hüllkörper aufweisen. Die Hüllkörper besitzen jeweils ein Lumen, das in ein gemeinsames Lumen im zum Verbleib außerhalb des Körpers vorgesehenen Abschnitt des Trokars mündet, Durch einen solchen Trokar sind Elektrodennadeln mit mehreren Schäften in das Körpergewebe einführbar. Vorteilhafterweise sind die Hüllkörper getrennt voneinander gegen den jeweiligen, sich durch sie erstreckenden Schaft verschiebbar.

Es zeigt sich, dass durch die Anwendung unterschiedlicher aktiver Längen der aus den Hüllkörpern herausragenden Schäfte bei näherungsweise achsenparallel eingebrachten Elektroden die erreichte thermische Destruktionszone in weiten Bereichen modelliert werden kann. Somit lässt sich auch bei komplexen Tumorgeometrien eine optimale Anpassung der Destruktionszone erzielen.

Zwar ist in den mit Bezug auf die Ausführungsbeispiele dargestellten Applikationsanordnungen eine Verschiebeeinrichtung zum Verschieben des Hüllkörpers relativ zum Schaft der Elektrodennadel beschrieben, die Rastpositionen aufweist, jedoch kann auch eine Verschiebevorrichtung vorhanden sein, mit der sich eine stufenlose Verschiebung und Arretierung des Hüllkörpers relativ zum Schaft ermöglichen lässt. Dabei ist beispielsweise an eine im Trokar vorgesehene Fixierschraube zu denken, mit der der Hüllkörper am Schaft in jeder Relativposition zueinander sicher fixiert werden kann.

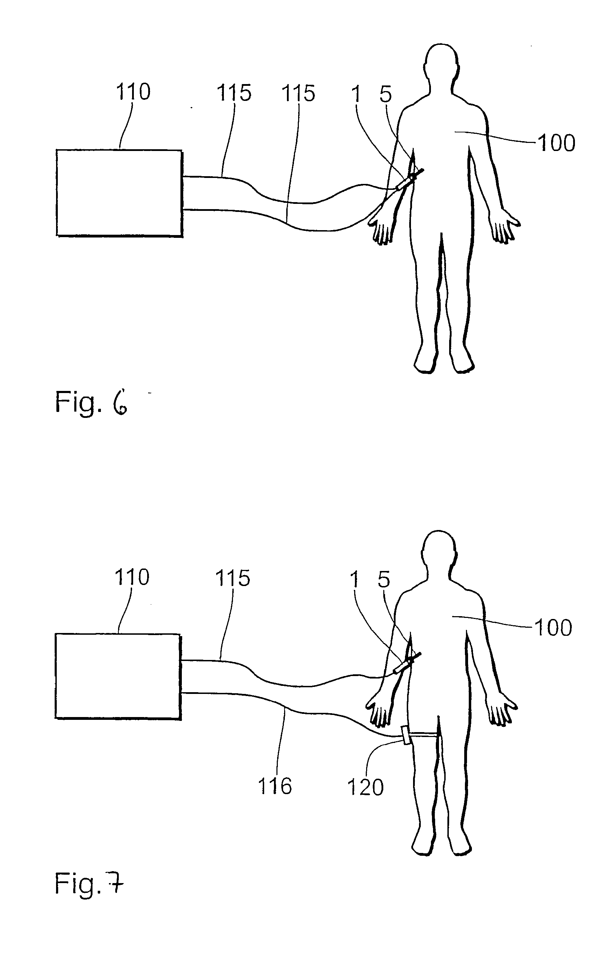

Eine erste Behandlungskonfiguration unter Verwendung der erfindungsgemäßen Applikationsanordnung ist schematisch in Figur 6 dargestellt. Eine im bipolaren Modus betriebene Elektrodennadel 1 ist durch einen in das Körpergewebe eines Patienten 100 eingebrachten Trokar 5 in einen zu verödenden Bereich des Körpers eingeführt und über zwei Leitungen 115 mit einem Hochfrequenzgenerator 110 verbunden. Der Hochfrequenzgenerator 110 stellt den zum elektrothermischen Veröden nötigen Hochfrequenzstrom zur Verfügung, der durch einen die Kabel 115, die Elektrodennadel 1 sowie das Körpergewebe umfassenden Stromkreis fließt. Der Hochfrequenzstrom fließt dabei zwischen den Elektroden der Elektrodennadel 1 durch das zu verödende Körpergewebe.

Eine zweite Behandlungskonfiguration unter Verwendung der erfindungsgemäßen Applikationsanordnung ist schematisch in Figur 7 dargestellt. Eine im monopolaren Modus betriebene Elektrodennadel 1 ist durch einen in das Körpergewebe eines Patienten 100 eingebrachten Trokar 5 in einen zu verödenden Bereich des Körpers eingeführt und über eine Leitung 115 mit einem Hochfrequenzgenerator 110 verbunden. Außerdem ist eine über eine Leitung 116 ebenfalls mit dem Hochfrequenzgenerator 110 verbundene Neutralelektrode 120 außen am Körper des Patienten 100 befestigt. Der Hochfrequenzgenerator 110 stellt den zum elektrothermischen Veröden nötigen Hochfrequenzstrom zur Verfügung, der durch einen das Kabel 115, das Kabel 116, die Elektrodennadel 1, die Neutralelektrode 120 sowie das Körpergewebe umfassenden Stromkreis fließt. Der Hochfrequenzstrom fließt dabei zwischen der Elektrodennadel 1 und der Neutralelektrode durch das zu verödende Körpergewebe.

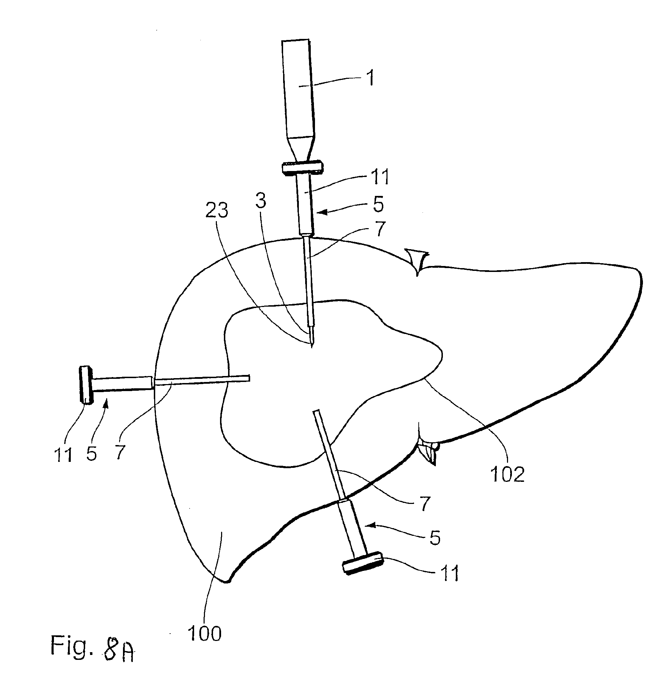

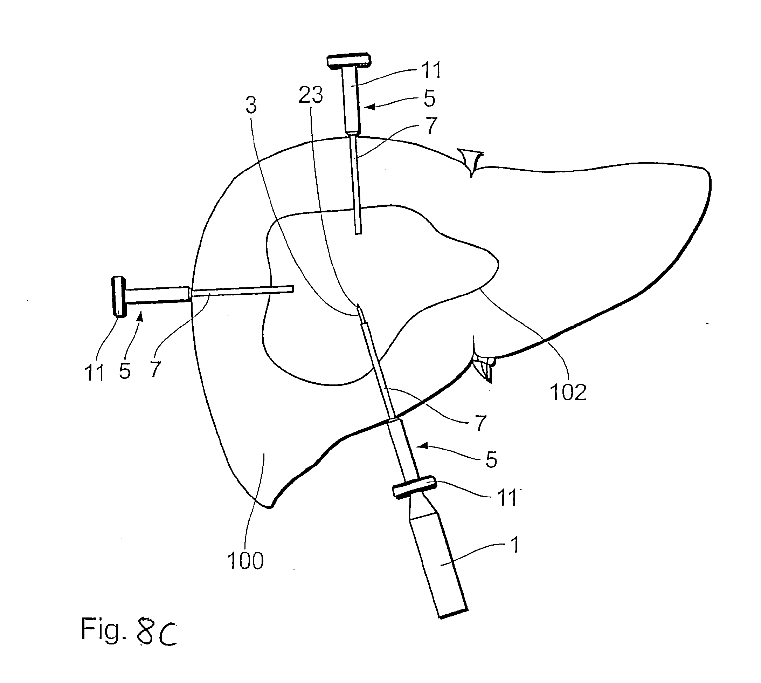

Mit Bezug auf die Figuren 8A bis 8C wird nun als ein Anwendungsbeispiel für die erfindungsgemäße Applikationsanordnung ein Volumenbehandlungsverfahren beschrieben. Die Volumenbehandlung dient zur Behandlung von pathogenem Tumorgewebe, vor allem in inneren Organen.

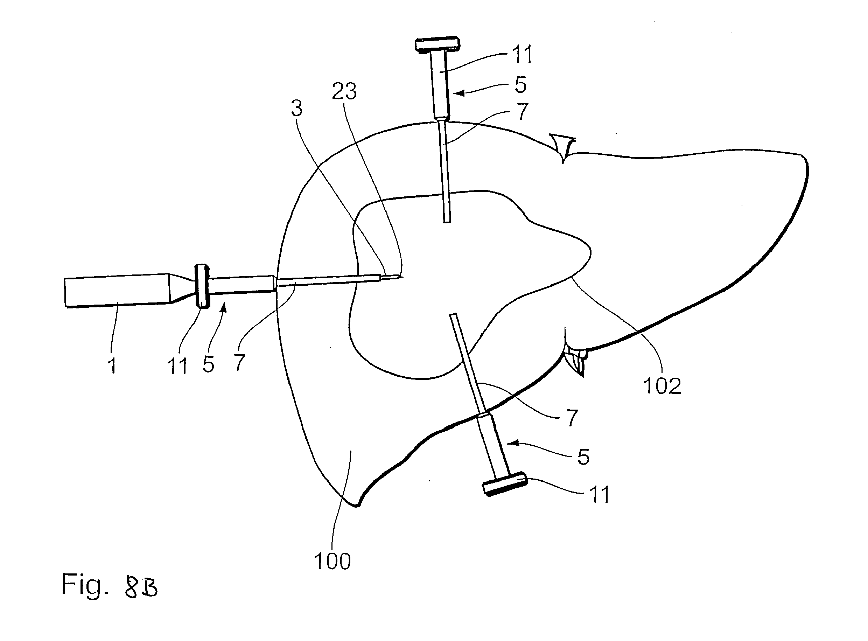

Die Figuren 8A, B und C zeigen schematisch einen Teil des Körpergewebes 100 eines Patienten und ein darin befindliches Tumorgewebe 102. Zur Durchführung des Volumenbehandlungsverfahrens wird eine Anzahl Trokare 5 derart in den Körper des Patienten eingeführt, das die distalen Enden ihrer zum Einführen in den Körper vorgesehenen Abschnitte, d.h. der Hüllkörper 7, in das Tumorgewebe 102 hineinreichen oder an dieses heranreichen.

Zum Einführen eines Trokars 5 wird eine Elektrodennadel 1 mit einer Spitze 23 am distalen Ende ihres Schaftes 3 in den Trokar 5 eingeführt und diese Kombination ins Körpergewebe 100 eingebracht, wobei die Spitze 23 zum Punktieren dient. Auf diese Weise werden alle Trokare 5 gesetzt.

Zum Zerstören des Tumorgewebes 102 wird zudem eine in den Figuren 8A bis 8C nicht dargestellte Neutralelektrode am Körper des Patienten angebracht, die als Gegenelektrode zum Schaft 3, oder besser gesagt der Schaftelektrode dient. Bei Anlegen einer Hochfrequenzspannung an die Elektrodennadel 1 fließt dann ein Strom zwischen dem aus dem distalen Ende des im Körpergewebe 100 befindlichen Hüllkörpers 7 herausragenden Abschnitt des Schaftes 3 und der Neutralelektrode. Damit wird eine Zerstörung des um den aktiven Schaft 3 herum befindlichen Tumorgewebes erreicht.

Nach einer gewissen Zeit oder wenn ein bestimmter Destruktionsgrad des Tumorgewebes erreicht ist, wird die Zuführung der Hochfrequenzspannung unterbrochen, die Elektrodennadel 1 aus dem in Figur 9A dargestellten Trokar 5 herausgezogen und in den in Figur 8B dargestellten Trokar 5 eingeführt. Dort wird die Applikation der Hochfrequenzspannung wiederholt, um so einen anderen Abschnitt des Tumorgewebes 102 zu zerstören. Dasselbe wiederholt sich dann, wie in Figur 8C dargestellt, bei einem weiteren in das Körpergewebe 100 eingeführten Trokar 5.

Es können entweder alle in den Figuren 8A bis C dargestellten Trokare 5 vor der ersten Applikation des Hochfrequenzstromes eingeführt werden, oder es kann alternativ jeder Trokar unmittelbar vor dem ersten Applizieren des Hochfrequenzstroms an einer bestimmten Stelle eingeführt werden, d.h. die in den Figuren 8B und C zum Applizieren des Hochfrequenzstromes verwendeten Trokare 5 werden erst unmittelbar vor dem jeweiligen Applikationsschritt eingeführt. Mit jedem Applizieren von Hochfrequenzstrom an einer noch nicht behandelten Stelle wird dabei ein neuer Trokar eingeführt, so lange, bis alle Trokare gesetzt sind und die Applikation nur noch an zuvor bereits behandelten Stellen erfolgt, an denen sich schon Trokare befindet.

Nach dem Applizieren des Hochfrequenzstroms verbleiben die Trokare 5 im zu behandelnden Körperbereich. Sie verhindern das Verschleppen von Tumorzellen beim Herausziehen der Nadel und dienen zum Zuführen von Medikamenten in das Therapievolumen, wie beispielsweise Schmerzmittel oder Chemotherapeutika. Außerdem können im Rahmen einer Feinnadelbiopsie dem Zielgewebe, also dem Tumorgewebe, Gewebeproben entnommen werden, um eine histologische Analyse durchzuführen. Nach dem Wiedereinführen der Elektrodennadel 1 in einen der Trokare 5 kann dann die Applikation der Hochfrequenzspannung wiederholt werden. Dabei kann jedes Mal die Länge des aktiven Bereichs des Schaftes 3 den klinischen Anforderungen entsprechend eingestellt werden.

Sobald die Behandlung insgesamt abgeschlossen ist, werden die Trokare 5 wieder aus dem Körpergewebe 100 entfernt.

Alternativ können auch gleichzeitig Elektrodennadeln 1 in alle in den Figuren 8A bis 8C dargestellten Trokare 5 eingeführt sein und gleichzeitig betrieben werden. Die Elektrodennadeln können 1 können dabei z.B. auf einem einheitlichen Potential liegen, wobei der Strom dann zu einer oder mehreren Neutralelektroden fließt (monopolarer Betrieb). Alternativ ist auch ein multipolarer Betrieb, also einer, bei dem die Elektroden mit unterschiedlichen Potentialen betrieben werden, möglich. Der gleichzeitige Betrieb der Elektrodennadeln 1 ermöglicht nach dem Ssuperpositionsprinzip eine Effizienzsteigerung, die das Behandeln großer Tumore ermöglicht.

In den Figuren 8A bis 8C sind jeweils 3 Trokare dargestellt. Die Zahle der in der Behandlung verwendeten Trokare ist jedoch nicht auf drei festgelegt, vielmehr kann sie der Art der Behandlung sowie der Art und/oder der Größe des Tumors angepasst werden.

## Patentansprüche

1. Applikationsanordnung zum Applizieren eines Hochfrequenzstroms zur thermischen Verödung von Körpergewebe, umfassend:
- eine Elektrodennadel (1) mit einem elektrisch leitenden Schaft (3),
- mindestens einen den elektrisch leitenden Schaft (3) umgebenden und relativ zum Schaft (3) verschiebbaren isolierenden Hüllkörper (7) mit einem distalen Ende, aus dem der Schaft (3) ausfahrbar ist,
**dadurch gekennzeichnet, dass**
- an der Außenseite des isolierenden Hüllkörpers (7) eine Gegenelektrode (25) angeordnet ist.

2. Applikationsanordnung nach Anspruch 1 mit einer an der Außenseite des isolierenden Hüllkörpers (7) angeordneten Gegenelektrode (25).

3. Applikationsanordnung nach Anspruch 2, **dadurch gekennzeichnet,**
- **dass** die Applikationsanordnung zusätzlich mindestens einen Trokar (5) mit einem zum Einführen in Körpergewebe vorgesehenen Abschnitt, einem zum Verbleib außerhalb des Körpers vorgesehenen Abschnitt (11) sowie einem sich durch beide Abschnitte erstreckenden Lumen (13), durch das der Schaft (3) der Elektrodennadel (1) durch den Trokar (5) hindurchzuführen ist,
**dass** der Trokar (5) einen elektrischen Anschluss (31) zum Anschließen eines Hochfrequenzgenerators an die Gegenelektrode (25) aufweist.

4. Applikationsanordnung nach Anspruch 3, **dadurch gekennzeichnet, dass** der elektrische Anschluss (31) als Steckkontakt ausgebildet ist, zu dem ein Gegenstück an einem nicht zum Einbringen in das Körpergewebe vorgesehen Teil der Elektrodennadel (1) existiert.

5. Applikationsanordnung nach Anspruch 4, **dadurch gekennzeichnet, dass** der Steckkontakt und das Gegenstück derart ausgestaltet und zueinander angeordnet sind, dass die Verbindung des Steckkontaktes mit dem Gegenstück beim Einführen der Elektrodennadel (1) in den Trokar (5) selbständig zustande kommt.

6. Applikationsanordnung nach Anspruch 3, **gekennzeichnet durch** eine Verschiebeeinrichtung (15) zum Verschieben des Hüllkörpers (7) relativ zum Schaft (3) derart, dass die Länge des aus dem distalen Ende des Hüllkörpers (7) herausragenden Teils des Schaftes (3) einstellbar ist.

7. Applikationsanordnung nach Anspruch 6, **gekennzeichnet durch** einen Klemm- oder Schraubmechanismus (17, 18, 19) zum Arretieren der Elektrodennadel (1) relativ zum Hüllkörper (7).

8. Applikationsanordnung nach Anspruch 7, **dadurch gekennzeichnet, dass** der Klemm- oder Schraubmechanismus (17, 18, 19) Markierungen (19) umfasst, denen sich die Länge des aus dem distalen Ende des Hüllkörpers (7) herausragenden Teils des Schaftes (3) bei in den Körper eingeführtem Schaft (3) entnehmen lässt.

9. Applikationsanordnung nach einem der Ansprüche 6 bis 8, **dadurch gekennzeichnet, dass** am zum Verbleib außerhalb des Körpers vorgesehenen Abschnitt (11) des Trokars (5) und der Elektrodennadel (1) eine Innen- und Außengewindekombination vorhanden ist, deren Gewinde derart ausgebildet und zueinander angeordnet sind, dass durch verdrehen der Elektrodennadel (1) gegen den zum Verbleib außerhalb des Körpers vorgesehenen Abschnitt (11) des Trokars (5) der Hüllkörper (7) relativ zum Schaft (3) axial zu verschieben ist.

10. Applikationsanordnung nach einem der Ansprüche 3 bis 9, **dadurch gekennzeichnet, dass** der Hüllkörper (7) den Schaft (3) eng umschließt.

11. Applikationsanordnung nach einem der Ansprüche 3 bis 9, **dadurch gekennzeichnet, dass** zwischen dem Schaft (3) und der Innenseite des Hüllkörpers (7) ein Spalt (21; 21') vorhanden ist.

12. Applikationsanordnung nach Anspruch 11, **dadurch gekennzeichnet, dass** die Abmessung des Spaltes (21, 21') derart gewählt ist, dass ein bestimmter Flüssigkeitsdruck überschritten werden muss, damit Flüssigkeit durch den Spalt fließen kann.

13. Applikationsvorrichtung nach Anspruch 11 oder 12, **dadurch gekennzeichnet, dass** am zum Verbleib außerhalb des Körpers vorgesehenen Abschnitt (11) des Trokars (5) eine Flüssigkeitszuführung zum Zuführen von Fluiden in den Spalt (21) angeordnet ist.

14. Applikationsanordnung nach einem der Ansprüche 3 bis 13, **dadurch gekennzeichnet, dass** der Schaft (3) an seinem distalen Ende mit einer Spitze (23) versehen ist.

15. Applikationsanordnung nach Anspruch 14, **dadurch gekennzeichnet, dass** der Hüllkörper (7) an seinem distalen Ende eine Fase, aufweist.

16. Applikationsanordnung nach einem der Ansprüche 3 bis 15, **dadurch gekennzeichnet, dass** der zum Einführen in Körpergewebe vorgesehene Abschnitt des Trokars (5) ein Material umfasst, das ihn in einer computertomographischen oder kernspintomographischen Aufnahme sichtbar macht.
